(19) Europäisches Patentamt
European Patent Office
Office européen des brevets

(11) **EP 1 852 178 B1**

(12) **EUROPEAN PATENT SPECIFICATION**

(45) Date of publication and mention
of the grant of the patent:
**16.08.2017   Bulletin 2017/33**

(21) Application number: **06712508.8**

(22) Date of filing: **27.01.2006**

(51) Int Cl.:
*B01J 13/04* (2006.01)          *A61K 9/10* (2006.01)
*A61K 9/127* (2006.01)        *A61K 31/7088* (2006.01)
*A61K 38/00* (2006.01)        *A61K 47/24* (2006.01)
*A61K 47/34* (2017.01)        *A61K 48/00* (2006.01)

(86) International application number:
**PCT/JP2006/301342**

(87) International publication number:
**WO 2006/080451 (03.08.2006 Gazette 2006/31)**

(54) **PROCESS FOR PRODUCING COATED FINE PARTICLE**

VERFAHREN ZUR HERSTELLUNG VON BESCHICHTETEN FEINEN TEILCHEN

PROCÉDÉ DE PRODUCTION D'UNE PARTICULE FINE ENROBÉE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **28.01.2005   JP 2005022240**

(43) Date of publication of application:
**07.11.2007   Bulletin 2007/45**

(73) Proprietor: **Kyowa Hakko Kirin Co., Ltd.
Tokyo 100-8185 (JP)**

(72) Inventors:
• **YAGI, Nobuhiro
  Shizuoka 411-8731 (JP)**
• **TOKUDA, Takuya
  Shizuoka 411-8731 (JP)**
• **NAKAKURA, Masashi
  Sakai-shi,
  Osaka 590-8554 (JP)**
• **KATO, Yasuki
  Susono-shi,
  Shizuoka 410-1115 (JP)**

(74) Representative: **Teipel, Stephan et al
Lederer & Keller
Patentanwälte Partnerschaft mbB
Unsöldstrasse 2
80538 München (DE)**

(56) References cited:
**EP-A1- 1 728 550          EP-A1- 1 731 173
WO-A1-02/28367          JP-A- 03 169 808
KR-A- 20030 041 999**

**Description**

Technical Field

The present invention relates to a method of producing coated fine particles.

Background Art

Heretofore, a lot of techniques related to methods of producing coated fine particles have been disclosed, for pharmaceutical products, foods, agrochemicals, drugs for animals and the like. The coating of fine particles (fine particles to be coated) with coating layer component(s) is carried out for imparting a function to fine particles such as to inhibit an effect given by an external factor, or to selectively receive an effect given by an external factor as a trigger causing change in the fine particles by the effect. As one of them, a method of coating fine particles with a lipid membrane in a liquid has been reported (see Patent document 1). In the method, fine particles are coated with a lipid membrane by reducing the ratio of a polar organic solvent in an aqueous solution comprising the polar organic solvent in which fine particles are dispersed and a lipid is dissolved, the coating is carried out in the liquid, and for example, coated fine particles with a size suitable for such as fine particles for intravenous injection and the like is produced very efficiently.

Methods of coating a micelle particle with a lipid membrane in a liquid have been reported (see, Patent Documents 2 and 3). In the method of Patent Document 2, for example, a cationic lipid is dissolved in chloroform beforehand, an aqueous solution of an oligodeoxynucleotide (ODN) and methanol are added to and mixed with the chloroform solution, the obtained mixture is centrifuged to introduce a cationic lipid/ODN complex into the chloroform layer, and the chloroform layer is isolated. Then, a polyethylene glycol-modified phospholipid, a neutral lipid, and water are added to the chloroform layer to form a water-in-oil (W/O) emulsion, and the obtained emulsion is subjected to reverse phase evaporation to produce an ODN-comprising liposome. On the other hand, in the method of Patent Document 3, for example, a complex (a micelle) of a lipid component and plasmid or oligonucleotide DNA having a histidine/fusogenic peptide conjugate is formed in 10 to 90% ethanol. The liquid is diluted with water and mixed with liposome or lipid prepared in advance, to convert the micelle to liposome, and the liposome is subjected to dialysis and passed through a membrane, so that plasmid- or oligonucleotide-entrapped liposome is produced.

Patent Document 4 relates to a method for coating fine particles which comprises dispersing fine particles in an aqueous solution containing a polar solvent; adding a lipid dissolved in an aqueous solution comprising a polar solvent; and adding water to the resulting aqueous mixture in order to decrease the rate of the polar organic solvent in the aqueous mixture.

Patent Document 1: WO 02/28367
Patent Document 2: Published Japanese translation of a PCT international application No. 2002-508765
Patent Document 3: Published Japanese translation of a PCT international application No. 2003-535832
Patent Document 4: EP 1 323 415 A1

Disclosure of the Invention

Problems to be Solved by the Invention

The object of the present invention is to provide a method of easily producing coated fine particles in which core fine particles are coated with a coating layer component. Means for Solving the Problems

The present invention relates to the subject matter disclosed in present claims 1-14.

Effect of the Invention

According to the present invention, a simple method of producing coated fine particles in which core fine particles are coated with coating layer component(s) is provided. Further, according to the present invention, a method of producing coated fine particles in which a drug is efficiently and/or firmly eclcosed is provided.

Brief Description of the Drawing

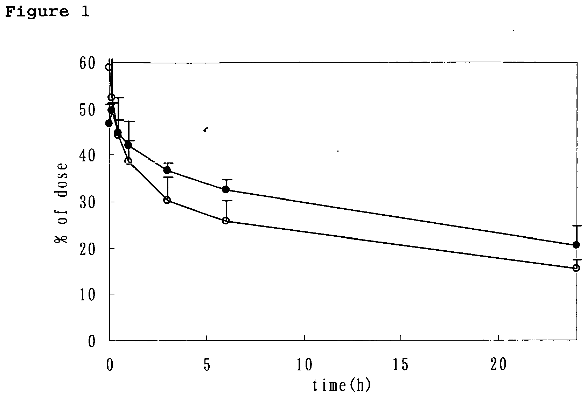

[Fig. 1] Blood kinetics of preparations obtained in Example 4 and Comparative Example 2 after administering them to rats are shown. The closed circles represent administration results of the preparation of Example 4, and the open circles represent administration results of the preparation of Comparative Example 2.

Best Mode for Carrying Out the Invention

In the present invention, the coated fine particles contain at least core fine particles and a coating layer, and are generated by coating the outer surface of the core fine particles with coating layer component(s) for forming the coating layer.

The core fine particles in the present invention are fine particles comprising as a constituent component, for example, a drug, a lipid assembly, liposome, emulsion particles, a polymer, a metal colloid, fine particles preparation or the like. Preferred examples include fine particles comprising at least a drug as a constituent component. The core fine particles in the present invention may contain, as a constituent component, a complex obtained by combining two or more of a drug, a lipid assembly, liposome, emulsion particles, a polymer, a metal colloid, fine particles preparation and the like, or may contain, as a constituent component, a complex obtained by combining a drug, a lipid assembly, liposome, emulsion particles, a polymer, a metal colloid, fine particles preparation or the like with another compound (such as a sugar, a lipid or an inorganic compound). Preferred examples include fine particles comprising, as a constituent component, a complex of a drug and one or more of a lipid assembly, liposome, emulsion particles, a polymer, a metal colloid and fine particles preparation. More preferred examples include fine particles comprising, as a constituent component, a complex of a drug and liposome.

The drug includes a drug which takes the form of fine particles in the solvent in the a liquid containing a polar organic solvent, a drug which forms a complex with another component constituting core fine particles and takes the form of fine particles in the solvent, and the like, and examples thereof include substances having a pharmacological activity among a protein, a peptide, a nucleic acid, a low-molecular compound, a saccharide, a polymer, a lipid compound, a metal compound and the like. Preferred examples include a nucleic acid and more preferred examples include one or more substance(s) selected from a gene, DNA, RNA, an oligonucleotide(ODN), plasmid, and siRNA.

Examples of the protein or the peptide include bradykinin, angiotensin, oxytocin, vasopressin, adrenocorticotropin, calcitonin, insulin, glucagon, cholecystokinin, β-endorphin, melanocyte inhibiting factor, melanocyte stimulating hormone, gastrin antagonist, neurotensin, somatostatin, brucine, cyclosporine, enkephalin, transferrin, Arg-Gly-Asp (RGD) peptide, thyroid hormone, growth hormone, gonadotropic hormone, luteinizing hormone, asparaginase, arginase, uricase, carboxypeptidase, glutaminase, superoxide dismutase, tissue plasminogen activator, streptokinase, interleukin, interferon, muramyl dipeptide, thymopoietin, granulocyte colony stimulating factor, granulocyte macrophage colony stimulating factor, erythropoietin, thrombopoietin, trypsin inhibitor, lysozyme, epidermal growth factor (EGF), insulin-like growth factor, nerve growth factor, platelet-derived growth factor, transforming growth factor, endothelial cell growth factor, fibroblast growth factor, glial growth factor, thymosin and specific antibody (such as anti-EGF receptor antibody) and the like.

Examples of the nucleic acid include ODN such as an antisense oligonucleotide and a sense oligonucleotide, a gene, DNA, RNA, plasmid, siRNA and the like. The nucleic acid includes derivatives in which an oxygen atom or the like contained in a phosphate moiety, an ester moiety, or the like in the nucleic acid structure has been substituted with another atom such as a sulfur atom. Incidentally, siRNA means a short double-stranded RNA.

Examples of the low-molecular compound include ε-aminocaproic acid, arginine hydrochloride, potassium L-aspartate, tranexamic acid, bleomycin sulfate, vincristine sulfate, cefazolin sodium, cephalothin sodium, citicoline, cytarabine, gentamicin sulfate, vancomycin hydrochloride, kanamycin sulfate, amikacin sulfate and the like.

Examples of the saccharide include sodium chondroitin sulfate, heparin sodium, dextran fluorescein and the like.

Examples of the polymer include sodium polyethylene sulfonate, a copolymer of divinyl ether with maleic anhydride (DIVEMA), a bonded product of a styrene-maleic anhydride copolymer with neocarzinostatin (SMANCS) and the like.

Examples of the lipid compound include vitamin D, vitamin E and the like.

Examples of the metal compound include cisplatin and the like.

The lipid assembly or the liposome is composed of, for example, lipid and/or a surfactant or the like. The lipid may be any of a simple lipid, a complex lipid and a derived lipid, and examples thereof include a phospholipid, a glyceroglycolipid, a sphingoglycolipid, a sphingoid, a sterol, a cationic lipid, and the like, and preferred examples include a phospholipid, a cationic lipid, and the like. Further, examples of the lipid also include surfactants (having the same definition as the surfactant described below), a polymer (having the same definition as the polymer described below, specifically dextran, etc.), and lipid derivative such as a polyoxyethylene derivative (specifically, polyethylene glycol, etc.), and preferred examples include a polyethylene glycolated lipid. Examples of the surfactant include a nonionic surfactant, an anionic surfactant, a cationic surfactant, a zwitterionic surfactant and the like.

Examples of the phospholipid include natural and synthetic phospholipids such as phosphatidylcholine (specifically, soybean phosphatidylcholine, egg yolk phosphatidylcholine (EPC), distearoyl phosphatidylcholine, dipalmitoyl phosphatidylcholine, dimyristoyl phosphatidylcholine, dioleoyl phosphatidylcholine, etc.), phosphatidylethanolamine (specifically, distearoyl phosphatidylethanolamine (DSPE), dipalmitoyl phosphatidylethanolamine, dioleoyl phosphatidylethanolamine, etc.), glycerophospholipid (specifically, phosphatidylserine, phosphatidic acid, phosphatidylglycerol, phosphatidylinositol, lysophosphatidylcholine, etc.) sphingophospholipid (specifically sphingomyelin, ceramide phosphoethanolamine, ceramide phosphoglycerol, ceramide phosphoglycerophosphate, etc.) glycerophosphono lipid, sphingophosphonolipid, natural lecithin (specifically, egg yolk lecithin, soybean lecithin, etc.) and hydrogenated phospholipid (specifically hydrogenated phosphatidylcholine, etc.).

Examples of the glyceroglycolipid include sulfoxyribosyl glyceride, diglycosyl diglyceride, digalactosyl diglyceride, galactosyl diglyceride, glycosyl diglyceride and the like.

Examples of the sphingoglycolipid include galactosyl cerebroside, lactosyl cerebroside, ganglioside and the like.

Examples of the sphingoid include sphingan, icosasphingan, sphingosine, a derivative thereof and the like. Examples of the derivative thereof include those in which -$NH_2$ of sphingan, icosasphingan, sphingosine or the like is replaced with -$NHCO(CH_2)_xCH_3$ (in the formula, x represents an integer of 0 to 18, in particular, 6, 12 or 18 is preferred) and the like.

Examples of the sterol include cholesterol, dihydrocholesterol, lanosterol, β-sitosterol, campesterol, stigmasterol, brassicasterol, ergocasterol, fucosterol, 3β-[N-(N'N'-dimethylaminoethyl)carbamoyl cholesterol (DC-Chol) and the like.

Examples of the cationic lipid include N-[1-(2,3-dioleoylpropyl)]-N,N,N-trimethylammonium chloride (DOTAP), N-[1-(2,3-dioleoylpropyl)]-N,N- dimethylamine (DODAP), N-[1-(2,3-dioleyloxypropyl)-N,N,N-trimethylammonium chloride (DOTMA), 2,3-dioleyloxy-N-[2-(sperminecarboxyamido)ethyl]-N,N-dimethyl-1-propanaminium trifluoroacetate (DOSPA), N-[1-(2,3-ditetradecyloxypropyl)]-N,N-dimethyl-N-hydroxyethylammonium bromide (DMRIE), N-[1-(2,3-dioleyloxypropyl)]-N,N-dimethyl-N-hydroxyethylammonium bromide (DORIE) and the like.

Examples of the nonionic surfactants include polyoxyethylene sorbitan monooleate (specifically, Polysorbate 80, etc.), polyoxyethylene polyoxypropylene glycol (specifically, Pluronic F68, etc.), a sorbitan fatty acid (specifically, sorbitan monolaurate, sorbitan monooleate, etc.), a polyoxyethylene derivative (specifically, polyoxyethylene hydrogenated castor oil 60, polyoxyethylene lauryl alcohol, etc.), a glycerol fatty acid ester and the like.

Examples of the anionic surfactants include acylsarcosine, sodium alkylsulfate, alkylbenzene sulfonate, a sodium fatty acid having 7 to 22 carbon atoms and the like. Specific examples include sodium dodecyl sulfate, sodium lauryl sulfate, sodium cholate, sodium deoxycholate, sodium taurodeoxycholate and the like.

Examples of the cationic surfactants include an alkylamine salt, an acylamine salt, a quaternary ammonium salt, an amine derivative and the like. Specific examples include benzalkonium chloride, an acylaminoethyldiethylamine salt, an N-alkylpolyalkylpolyamine salt, a polyethylene polyamide of fatty acid, cetyltrimethylammonium bromide, dodecyltrimethylammonium bromide, alkylpolyoxyethyleneamine, N-alkylaminopropylamine, a triethanolamine fatty acid ester and the like.

Examples of the zwitterionic surfactants include 3-[(3-cholamidopropyl)dimethylammonio]-1-propane sulfonate, N-tetradecyl-N,N-dimethyl-3-ammonio-1-propane sulfonate and the like.

In the liposome, these lipid and surfactants are used alone or in combination, and preferably they are used in combination. As the combination in the case where they are used in combination, for example, a combination of two or more components selected from a hydrogenated soybean phosphatidylcholine, a polyethylene glycolated phospholipid and cholesterol, a combination of two or more components selected from distearoyl phosphatidylcholine, a polyethylene glycolated phospholipid and cholesterol, a combination of EPC and DOTAP, a combination of EPC, DOTAP and a polyethylene glycolated phospholipid, a combination of EPC, DOTAP, cholesterol and a polyethylene glycolated phospholipid, and the like can be exemplified.

Further, the liposome may contain a membrane stabilizer such as a sterol including cholesterol, an antioxidant such as tocopherol or the like, as needed.

Examples of the lipid assembly include a spherical micelle, a spherical reversed micelle, a sausage-shaped micelle, a sausage-shaped reversed micelle, a plate-shaped micelle, a plate-shaped reversed micelle, hexagonal I, hexagonal II, an associated product comprising two or more lipid molecules, and the like.

Examples of the emulsion particles include oil-in-water (o/w) emulsion particles such as a fat emulsion, an emulsion composed of a nonionic surfactant and soybean oil, lipid emulsion and lipid nanosphere, water-in-oil-in-water (w/o/w) emulsion particles and the like.

Examples of the polymer include natural polymers such as albumin, dextran, chitosan, dextran sulfate and DNA, synthetic polymers such as poly-L-lysine, polyethyleneimine, polyaspartic acid, a copolymer of styrene with maleic acid, a copolymer of isopropylacrylamide with acrylpyrrolidone, PEG-modified dendrimer, polylactic acid, polylactic acid polyglycolic acid and polyethylene glycolated polylactic acid, a salt thereof and the like.

Here, the salt of the polymer includes, for example, a metal salt, an ammonium salt, an acid addition salt, an organic amine addition salt, an amino acid addition salt and the like. Examples of the metal salt include alkali metal salts such as a lithium salt, a sodium salt and a potassium salt, alkaline earth metal salts such as a magnesium salt and a calcium salt, an aluminum salt, a zinc salt and the like. Examples of the ammonium salt include salts of ammonium, tetramethylammonium and the like. Examples of the acid addition salt include inorganates such as a hydrochlorate, a sulfate, a nitrate and a phosphate, and organates such as an acetate, a maleate, a fumarate and a citrate. Examples of the organic amine addition salt include addition salts of morpholine, piperidine and the like, and examples of the amino acid addition salt include addition salts of glycine, phenylalanine, aspartic acid, glutamic acid, lysine and the like.

Examples of the metal colloid include metal colloids including gold, silver, platinum, copper, rhodium, silica, calcium, aluminum, iron, indium, cadmium, barium, lead and the like.

Examples of the fine particles preparation include a microsphere, a microcapsule, a nanocrystal, lipid nanoparticles, a polymeric micelle and the like.

The core fine particles produced by the method according to the present invention preferably contain a lipid derivative or a fatty acid derivative of one or more substance(s) selected from, for example, sugars, peptides, nucleic acids and water-soluble polymers or a surfactant or the like. The lipid derivative or the fatty acid derivative of one or more substance(s) selected from sugars, peptides, nucleic acids and water-soluble polymers or the surfactant may be contained in the constituent components of the core fine particles or may be used by adding it to the constituent components of the core fine particles.

Preferred examples of the lipid derivative or the fatty acid derivative of one or more substance (s) selected from sugars, peptides, nucleic acids and water-soluble polymers or the surfactant include a glycolipid or a lipid derivative or a fatty acid derivative of a water-soluble polymer, and more preferred examples thereof include a lipid derivative or a fatty acid derivative of a water-soluble polymer. The lipid derivative or the fatty acid derivative of one or more substance (s) selected from sugars, peptides, nucleic acids and water-soluble polymers or the surfactant is preferably a substance having a dual character that a part of the molecule has a property of binding to another constituent component of the core fine particles due to, for example, hydrophobic affinity, electrostatic interaction or the like, and other part has a property of binding to a solvent used in the production of the core fine particles due to, for example, hydrophilic affinity, electrostatic interaction or the like.

Examples of the lipid derivative or the fatty acid derivative of a sugar, a peptide or a nucleic acid include those comprising a sugar such as sucrose, sorbitol or lactose, a peptide such as a casein-derived peptide, an egg white-derived peptide, a soybean-derived peptide or glutathione, a nucleic acid such as DNA, RNA, plasmid, siRNA or ODN, and any of the lipid illustrated in the above-mentioned definition of the core fine particles or a fatty acid such as stearic acid, palmitic acid, myristic acid or lauric acid bonded to each other and the like. Examples of the lipid derivative or the fatty acid derivative of a sugar include the glycolipids such as glyceroglycolipids or sphingoglycolipids illustrated in the above-mentioned definition of the core fine particles and the like.

Examples of the lipid derivative or the fatty acid derivative of a water-soluble polymer include those comprising polyethylene glycol, polyglycerol, polyethyleneimine, polyvinyl alcohol, polyacrylic acid, polyacrylamide, oligosaccharide, dextrin, a water-soluble cellulose, dextran, chondroitin sulfate, polyglycerol, chitosan, polyvinylpyrrolidone, polyaspartate amide, poly-L-lysine, mannan, pullulan, oligoglycerol or the like or a derivative thereof and any of the lipid illustrated in the above-mentioned definition of the core fine particles or a fatty acid such as stearic acid, palmitic acid, myristic acid or lauric acid bonded to each other and the like. More preferably, a lipid derivative or a fatty acid derivative of a polyethylene glycol derivative or a polyglycerol derivative can be exemplified, and further more preferably, a lipid derivative or a fatty acid derivative of a polyethylene glycol derivative can be exemplified.

Examples of the lipid derivative or the fatty acid derivative of a polyethylene glycol derivative include a polyethylene glycolated lipid [specifically, polyethylene glycol phosphatidyl ethanolamine (more specifically, 1,2-distearoyl-sn-glycero-3-phosphoethanolamine-N-[methoxy (polyethylene glycol)-2000] (PEG-DSPE) and the like), polyoxyethylene hydrogenated castor oil 60, Cremophor EL and the like], a polyethylene glycol sorbitan fatty acid ester (specifically, polyoxyethylene sorbitan monooleate and the like), a polyethylene glycol fatty acid ester and the like, and more preferred examples include a polyethylene glycolated lipid.

Examples of the lipid derivative or the fatty acid derivative of a polyglycerol derivative include a polyglycerolated lipid (specifically, polyglycerol phosphatidyl ethanolamine and the like), a polyglycerol fatty acid ester and the like, and more preferred examples include a polyglycerolated lipid.

Examples of the surfactant include the surfactants illustrated in the above-mentioned definition of the core fine particles, a polyethylene glycol alkyl ether and the like, and preferred examples thereof include polyoxyethylene polyoxypropylene glycol, a glycerol fatty acid ester, a polyethylene glycol alkyl ether and the like.

In a case where the core fine particles contains the complex of a drug and one or more constituent component selected from the group consisting of a lipid assembly, liposome, emulsion particles, a polymer, a metal colloid, and fine particles preparation, it is preferred that the component has an electrostatic charge opposite to that of the drug. The electrostatic charge of the component, opposite to that of the drug, may be a charge, a surface polarization, etc. that generates an electrostatic attraction together with an intramolecular charge, an intramolecular polarization, etc. of the drug. In order for a lipid assembly, liposome, emulsion particles, a polymer, a metal colloid, and fine particles preparation to have the electrostatic charge opposite to that of the drug, a lipid assembly, liposome, emulsion particles, a polymer, a metal colloid, and fine particles preparation preferably comprises a charged substance having the electrostatic charge opposite to that of the drug, and more preferably comprises a lipid having the electrostatic charge opposite to that of the drug (the above-mentioned cationic lipid or the anionic lipid to be hereinafter described).

The charged substance having the electrostatic charge opposite to that of the drug such as a lipid assembly, liposome, emulsion particles, a polymer, a metal colloid, and fine particles preparations may be classified into a cationic substance exhibiting a cationic property and an anionic substance exhibiting an anionic property. However, even if it is a zwitterionic substance having both of cationic group and anionic group, the relative electronegativity changes depending on the pH, bonding with another substance or the like, and it can be classified into a cationic substance or an anionic substance depending on the conditions. Such a charged substance may be used, as a constituent component, of the core fine particles or may be used by adding it to the constituent component of the core fine particles.

Examples of the cationic substance include the cationic substances among those illustrated in the above-mentioned definition of the core fine particles (specifically, a cationic lipid (having the same definition as above), a cationic sterol, a cationic surfactants (having the same definition as above), a cationic polymer and the like), a protein or a peptide with which a complex can be formed at a pH equal to or less than an isoelectric point, and the like.

Examples of the cationic sterol include DC-Chol and the like.

Examples of the cationic polymer include poly-L-lysine, polyethyleneimine, polyfect, chitosan and the like.

The protein or the peptide with which a complex can be formed at a pH equal to or less than an isoelectric point is not particularly limited as long as it is a protein or a peptide with which a complex can be formed at a pH equal to or less than the isoelectric point of the substance. Examples thereof include albumin, orosomucoid, globulin, fibrinogen, pepsin, ribonuclease T1 and the like.

Examples of the anionic substance include the anionic substances among those illustrated in the above-mentioned definition of the core fine particles [specifically, an anionic lipid, an anionic surfactants (having the same definition as above), an anionic polymer and the like], a protein or a peptide, with which a complex can be formed at a pH equal to or greater than an isoelectric point, a nucleic acid and the like.

Examples of the anionic lipid include phosphatidylserine, phosphatidylglycerol, phosphatidylinositol, phosphatidic acid and the like.

Examples of the anionic polymer include polyaspartic acid, a copolymer of styrene with maleic acid, a copolymer of isopropylacrylamide with acrylpyrrolidone, PEG-modified dendrimer, polylactic acid, polylactic acid polyglycolic acid, polyethylene glycolated polylactic acid, dextran sulfate, sodium dextran sulfate, chondroitin sulfate, sodium chondroitin sulfate, hyaluronic acid, chondroitin, dertaman sulfate, heparan sulfate, heparin, ketaran sulfate, dextran fluorescein anionic and the like.

The protein or the peptide with which a complex can be formed at a pH equal to or greater than an isoelectric point is not particularly limited as long as it is a protein or a peptide with which a complex can be formed at a pH equal to or greater than the isoelectric point of the substance. Examples thereof include albumin, orosomucoid, globulin, fibrinogen, histone, protamine, ribonuclease, lysozyme and the like.

Examples of the nucleic acid as an anionic substance include DNA, RNA, plasmid, siRNA, ODN and the like. It may have any length and any sequence as long as it does not exhibit a physiological activity.

In a case where the core fine particles contain the complex of a drug and one or more constituent component selected from the group consisting of a lipid assembly, liposome, emulsion particles, a polymer, a metal colloid, and fine particles preparation, and a lipid assembly, liposome, emulsion particles, a polymer, a metal colloid or fine particles preparation has the electrostatic charge opposite to that of the drug, it is further preferred that the core fine particles contain an adhesion-competitive agent.

As the adhesion-competitive agent in the present invention, for example, a substance having the same electrostatic charge as that of the drug and the like can be exemplified, and a substance electrostatically adhered to a lipid assembly, liposome, emulsion particles, a polymer, a metal colloid or fine particles preparation which is a constituent component of the core fine particles, due to the electrostatic attraction to a cation or an anion by an electric charge in the molecule, intramolecular polarization or the like is included. Examples thereof include a lipid, surfactants, a nucleic acid, a protein, a peptide, a polymer and the like. Examples of the lipid, the surfactant, the nucleic acid, the protein, the peptide and the polymer include the cationic lipids, the anionic lipids, the cationic surfactants, the anionic surfactants, the nucleic acids, the proteins, the peptides, the cationic polymers and the anionic polymers illustrated in the above-mentioned definition of the charged substance and the like. Preferred examples include the cationic polymers and the anionic polymers illustrated in the above-mentioned definition of the charged substance and the like, and more preferred examples include one or more substance(s) selected from dextran sulfate, sodium dextran sulfate, chondroitin sulfate, sodium chondroitin sulfate, hyaluronic acid, chondroitin, dertaman sulfate, heparan sulfate, heparin, ketaran sulfate, dextran fluorescein anionic, poly-L-lysine, polyethyleneimine, polyfect, chitosan and the like. The adhesion-competitive agent preferably is electrostatically adhered to a lipid assembly, liposome, emulsion particles, a polymer, a metal colloid or fine particles preparation which is a constituent component of the core fine particles, and is preferably a substance with a size which does not allow the crosslinking formation to aggregate the lipid assembly, liposome, emulsion particles, a polymer, a metal colloid or fine particles preparation which is a constituent component of the core fine particles, even if the substance is adhered to the to a lipid assembly, liposome, emulsion particles, a polymer, a metal colloid or fine particles preparation, or a substance having a moiety in its molecule, which repels the adhesion of the lipid assembly, liposome, emulsion particles, the polymer, the metal colloid or fine particles preparation, thereby suppressing the aggregation of the lipid assembly, liposome, emulsion particles, the polymer, the metal colloid or fine particles preparation.

The core fine particles produced according to the method of the present invention can be produced by or in accordance with a known production method, and may be produced by any production method. For example, in the production of the core fine particles comprising, as a constituent component, liposome, which is one of the core fine particles, a known liposome preparation method can be applied. As the known liposome preparation method, for example, liposome preparation method by Bangham, et al. [see "Journal of Molecular Biology" (J. Mol. Biol.), vol. 13, pp. 238-252 (1965)], an ethanol injection method [see "Journal of Cell Biology" (J. Cell Biol.), vol. 66, pp. 621-634 (1975)], a French press method [see "FEBS Letters" (FEBS Lett.), vol. 99, pp. 210-214 (1979)], a freeze-thaw method [see "Archives of Biochemistry and Biophysics" (Arch. Biochem. Biophys.), vol. 212, pp. 186-194 (1981)], a reverse phase evaporation method [see "Proceedings of the National Academy of Science United States of America" (Proc. Natl. Acad. Sci. USA), vol. 75, pp. 4194-4198 (1978)], a pH gradient method (see, for example, Japanese Patent No. 2, 572, 554, Japanese Patent No. 2, 659, 136, etc.) and the like. As a solution for dispersing liposome in the production of the liposome, for example, water, an acid, an alkali, any of various buffers, a physiological saline solution, an amino acid infusion or the like can be used. Further, in the production of the liposome, it is also possible to add an antioxidant such as citric acid, ascorbic acid, cysteine or ethylenediamine tetraacetic acid (EDTA), an isotonic agent such as glycerol, glucose, sodium chloride or the like. Further, the liposome can be prepared by dissolving lipid or the like in, for example, an organic solvent such as ethanol, distilling off the solvent, adding a physiological saline solution or the like and stirring the mixture by shaking, thereby forming liposome.

Further, surface improvement of the liposome can be optionally carried out using, for example, a nonionic surfactants (having the same definition as above), a cationic surfactants (having the same definition as above), an anionic surfactants (having the same definition as above), a polymer, a polyoxyethylene derivative or the like, and such a surface-improving liposome is also used as a constituent component of the core fine particles in the present invention [see "Stealth Liposome", edited by D. D. Lasic and F. Martin, CRC Press Inc., USA, pp. 93-102 (1995)]. Examples of the polymer include dextran, pullulan, mannan, amylopectin, hydroxyethylstarch and the like. Examples of the polyoxyethylene derivative include Polysorbate 80, Pluronic F68, polyoxyethylene hydrogenated castor oil 60, polyoxyethylene lauryl alcohol, PEG-DSPE and the like. The surface improvement of the liposome can be employed as one of the methods of incorporating lipid derivative or a fatty acid derivative of one or more substance(s) selected from sugars, peptides, nucleic acids and water-soluble polymers or a surfactant in the core fine particles.

An average particle diameter of the liposome can be freely selected upon demand. Examples of a method of adjusting the average particle diameter include an extrusion method and a method in which a large multilamellar liposome vesicle (MLV) is mechanically pulverized (specifically using Manton-gaulin, a microfluidizer or the like) [see "Emulsion and Nanosuspensions for the Formulation of Poorly Soluble Drugs", edited by R. H. Muller, S. Benita and B. Bohm, Scientific Publishers, Stuttgart, Germany, pp. 267-294 (1998)] and the like.

In addition, the method of producing a complex obtained by combining two or more substances selected from, for example, a drug, a lipid assembly, liposome, emulsion particles, a polymer, a metal colloid, fine particles preparation and the like, which constitute the core fine particles (specific examples include a complex of liposome or a lipid assembly comprising a cationic lipid and a nucleic acid, a complex of a polymer comprising a cationic polymer such as poly-L-lysine and a nucleic acid, a complex of liposome or a lipid assembly comprising an anionic lipid such as phosphatidic acid and a protein, a complex of a polymer comprising an anionic polymer such as styrene-maleic acid and a protein, a complex of liposome or a lipid assembly comprising a cationic lipid and a protein, a complex of a polymer comprising a cationic polymer such as poly-L-lysine and a protein and the like) may be, for example, a production method in which a drug is simply mixed with a lipid assembly, liposome, a polymer or the like in water. At this time, a sieve step, a sterilization step or the like can be further added as needed. Further, it is also possible to perform the formation of the complex in any of various solvents such as acetone and ether. For example, a nucleic acid and a lipid are dissolved in an organic solvent such as ethanol, the solvent is distilled off, a physiological saline solution or the like is added thereto, and the mixture is stirred by shaking, whereby a nucleic acid complex can also be formed.

As for the size of the core fine particles in the present invention, an average particle diameter is preferably several nanometers to several hundreds of micrometers, more preferably 10 nm to 5 $\mu$m, further more preferably 50 nm to 300 nm, the most preferably 50 nm to 200 nm.

In the present invention, the coating layer component (s) are dispersed in the liquid C comprising the polar organic solvent under a controlled concentration of the polar organic solvent. Examples of the coating layer component(s) include a lipid, a surfactant, a polymer, and the like described in the above definition of the core fine particles. The coating layer component(s) preferably comprises one or more substance(s) selected from the group consisting of the lipid and surfactants described in the above definition of the core fine particles, more preferably comprises one or more substances selected from the group consisting of the lipids and surfactants for forming a coating layer of a lipid membrane, and further preferably comprises a neutral lipid among the lipids and surfactants. The neutral lipids include lipids other than the cationic lipids, cationic surfactants, anionic lipids, and anionic surfactants, among the lipids and surfactants described in the above definition of the core fine particles. More preferred examples of the neutral lipids include phospholipids, glycoglycerolipids, glycosphingolipids, and the like, further preferred examples thereof include phospholipids, and most preferred examples thereof include EPCs.

The combination of core fine particles with coating layer component (s) in the present invention is not particularly limited, however, a combination of the core fine particles which are fine particles comprising, as a constituent component, a complex of a drug and liposome with the coating layer component (s) which is a lipid and/or a surfactant is preferred. Coated fine particles in which the core fine particles are fine particles comprising, as a constituent component, liposome, the coating layer component (s) is a lipid and/or a surfactant and the coating layer is a lipid membrane is classified into liposome in a narrow sense based on its structure. Coated fine particles in which the core fine particles is other than fine particles comprising, as a constituent component, liposome, the coating layer component (s) is a lipid and/or a surfactant and the coating layer is a lipid membrane is classified into liposome in a wide sense. In the present invention, it is more preferred that the constituent component of the core fine particles and the coated fine particles are liposome.

It is preferred that the coating layer component(s) intramolecularly has a hydrophobic moiety with an affinity for the polar organic solvent used in the present invention, and is soluble in the polar organic solvent due to the hydrophobic moiety, and it is more preferred that the coating layer component(s) intramolecularly has a hydrophilic moiety in addition to the hydrophobic moiety. Further, the coating layer component (s) preferably forms an aggregate or micelle, which preferably has a size of 0.1 to 1000 nm, more preferably has a size of 0.3 to 500 nm. The size of the aggregate or micelle may be measured by examining whether it can pass through a membrane filter having a desired pore size.

In the present invention, the coating layer component (s) is considered to be soluble in the polar organic solvent in a case where the coating layer component (s) can be dissolved per se in the polar organic solvent, and in a case where the coating layer component (s) can be dissolved by using a solubilizing agent or the like in the polar organic solvent. It is preferred that the coating layer component(s) can be dissolved per se in the polar organic solvent.

Further, examples of the lipid to be used when the coating layer is a lipid membrane include a synthetic lipid and the like. Examples of the synthetic lipid include fluorinated phosphatidylcholine, fluorinated surfactants, dialkylammonium bromide and the like. These may be used alone or in combination with another lipid or the like. Further, when the coating layer is a lipid membrane, the coating layer component(s) preferably contains a lipid derivative, a fatty acid derivative or an aliphatic hydrocarbon derivative of a water-soluble substance, or the above-mentioned lipid derivative or the fatty acid derivative of one or more substance (s) selected from sugars, peptides, nucleic acids and water-soluble polymers or the surfactant, more preferably contains the above-mentioned lipid derivative or the fatty acid derivative of a water-soluble polymer, further more preferably contains the above-mentioned polyethylene glycolated phospholipid, and most preferably contains polyethylene glycol phosphatidyl ethanolamine.

Examples of the lipid derivative, fatty acid derivative or aliphatic hydrocarbon derivative of a water-soluble substance in the present invention include the above-mentioned lipid derivative or fatty acid derivative of one or more substance(s) selected from sugars, proteins, peptides, nucleic acids and water-soluble polymers or the aliphatic hydrocarbon derivative of one or more substance(s) selected from sugars, proteins, peptides, nucleic acids and water-soluble polymers, preferred examples thereof include a glycolipid and a lipid derivative or a fatty acid derivative of a water-soluble polymer, and more preferred examples thereof include a lipid derivative or a fatty acid derivative of a water-soluble polymer.

Examples of the aliphatic hydrocarbon derivative of a water-soluble substance include those comprising a water-soluble substance and, for example, a long-chain aliphatic alcohol, polyoxypropylene alkyl, an alcoholic residue of a glycerin fatty acid ester or the like bonded to each other.

Examples of the aliphatic hydrocarbon derivative of a sugar, a peptide or a nucleic acid include an aliphatic hydrocarbon derivative of a sugar such as sucrose, sorbitol or lactose, a peptide such as a casein-derived peptide, an egg white-derived peptide, a soybean-derived peptide or glutathione, a nucleic acid such as DNA, RNA, plasmid, siRNA or ODN.

Examples of the aliphatic hydrocarbon derivative of a water-soluble polymer include an aliphatic hydrocarbon derivative of polyethylene glycol, polyglycerol, polyethyleneimine, polyvinyl alcohol, polyacrylic acid, polyacrylamide, oligosaccharide, dextrin, a water-soluble cellulose, dextran, chondroitin sulfate, polyglycerol, chitosan, polyvinylpyrrolidone, polyaspartate amide, poly-L-lysine, mannan, pullulan, oligoglycerol or the like or a derivative thereof, and more preferred examples thereof include an aliphatic hydrocarbon derivative of a polyethylene glycol derivative or a polyglycerol derivative, and more preferred examples thereof include an aliphatic hydrocarbon derivative of a polyethylene glycol derivative.

The term "the core fine particles are dispersed" means that the core fine particles are suspended, emulsified or made into an emulsion, preferably suspended. A small part of the core fine particles may be deposited in the liquid C, while a large part thereof is dispersed. It is preferred that all core fine particles are dispersed in the liquid C. The term "the coating layer component(s) is dispersed" means that the coating layer component(s) forms an aggregate, micelle, or the like and is suspended, emulsified or made into an emulsion, preferably emulsified and made into an emulsion. A small part of the coating layer component (s) may be dissolved in the liquid C, while a large part thereof forms the aggregate, micelle, or the like and is emulsified or made into an emulsion. Further, a small part of the coating layer component(s) may be deposited in the liquid C, while a large part thereof forms the aggregate, micelle, or the like and is suspended, emulsified or made into an emulsion, preferably emulsified or made into an emulsion. There are no particular limitations on the preparation of the dispersion liquid Cas long as the coating layer component(s) is soluble in the polar organic solvent, and the polar organic solvent concentration of the dispersion liquid is such that the core fine particles are not dissolved and the coating layer component(s) is in the dispersed state in the liquid as disclosed in claim 1.

There are no particular limitations on the period of leaving or mixing liquid C, as long as the leaving or mixing step is not stopped immediately after the core fine particles and the coating layer component (s) are dispersed in the liquid comprising the polar organic solvent. The period may be selected depending on the types of the coating layer component(s) and the liquid comprising the polar organic solvent, and is preferably such that the yield of the produced coated fine particles is steady. The period is 3 seconds to 30 minutes.

The method according to the invention particularly comprises the steps of: preparing a liquid A comprising the polar organic solvent as described above in which the coating layer component (s) is dissolved; preparing a liquid B mixable with the liquid A, in which the core fine particles are dispersed and a polar organic solvent as described above is not contained or the polar organic solvent is contained in a ratio lower than that of the liquid A; and mixing the liquid A with the liquid B to prepare a liquid C as described above. There are no particular limitations on the preparation of the liquids A and B as long as the polar organic solvent concentration of the mixed liquid C is such that the core fine particles are not dissolved and the coating layer component (s) can be in the dispersed state. The liquid C is left or mixed for a period of 3 seconds to 30 minutes for coating the core fine particles with the coating layer component.

Examples of the polar organic solvents usable in the present invention include alcohols such as methanol, ethanol, n-propanol, 2-propanol, n-butanol, 2-butanol, and tert-butanol; glycols such as glycerin, ethylene glycol, and propylene glycol; polyalkylene glycols such as polyethylene glycol, polyoxyethylene hardened castor oil, and polyoxyethylene sorbitan fatty acid ester or ether; and the like. The polar organic solvent is preferably ethanol. Examples of solvents usable in the invention, other than the polar organic solvent, include water, liquid carbon dioxide, liquid hydrocarbons, halogenated carbons, halogenated hydrocarbons, and the like, and preferred among them is water. The liquid C may contain a buffer component, or the like.

The combination of the polar organic solvent and the solvent other than the polar organic solvent is preferably such that the solvents are mixable with each other, and may be selected based on the solubilities of the above-mentioned core fine particles and the above-mentioned coating layer component (s) in each solvent of the liquid comprising the polar organic solvent in each process, etc. The core fine particles preferably have a low solubility in the liquid comprising the polar organic solvent in each process, and preferably have a low solubility in each of the polar organic solvent and the solvent other than the polar organic solvent. The coating layer component(s) preferably has a high solubility in the polar organic solvent, and preferably has a low solubility in the solvent other than the polar organic solvent.

In a case where the core fine particles is soluble in the polar organic solvent, the solubility of the core fine particles in the polar organic solvent is preferably lower than that of the coating layer component. The term "the solubility of the core fine particles is low" means that each constituent component of the core fine particles has a low elutability in the solvent, and the solubility of each constituent component may be high as long as each constituent component has a low elutability due to binding between the components, etc.

There are no particular limitations on the ratio of the polar organic solvent in the liquid C, as long as the core fine particles and the coating layer component(s) can be dispersed. The polar organic solvent concentration of the liquid C may be selected depending on the types of the solvent, the core fine particles, and the coating layer component, etc. , and is 30 to 60 vol%, preferably 30 to 40 vol%.

It is preferred that the hydrophilic interaction is acted between the coating layer component (s) and the surface of the core fine particles in the dispersion liquid C. Thus, the core fine particles and the coating layer component(s) preferably have a hydrophilic moiety.

There are no particular limitations on the methods of mixing or dispersing in the above steps. Examples of the methods include those using an in-line mixer, a static mixer, a propeller mixer, a rotor/stator mixer, a turbine mixer, a saw blade, a colloid mill, a high-pressure homogenizer, or the like, and preferred examples thereof include those using an in-line mixer, a static mixer, a propeller mixer, a rotor/stator mixer, or a turbine mixer.

For example, the coated fine particles having the coating layer of the lipid membrane, according to the invention, may be preferably produced by the following steps.

(Step 1) A lipid and/or a surfactant which is a coating layer (components of the lipid membrane) are dissolved or dispersed in the polar organic solvent or an aqueous solution comprising the polar organic solvent. A lipid derivative or a fatty acid derivative of one or more substance(s) selected from saccharides, peptides, nucleic acids, and water-soluble polymers, or a surfactant (e.g. a polyethylene glycol-modified lipid derivative) may be further added to the liquid, and the amount thereof is not particularly limited.

(Step 2) The core fine particles are dispersed (suspended) in an aqueous solution.
(Step 3) The liquids obtained in the steps 1 and 2 are mixed.

The coated fine particles of the present invention can be produced in substantially the same manner as above regardless of the types of the core fine particles and the coating layer component.

In the method of the present invention for producing the coated fine particles, there are no particular limitations on the concentration of the core fine particles in the liquid C as long as the core fine particles can be coated with the coating layer as disclosed by claim 1. The core fine particles concentration is preferably 1 μg/mL to 1 g/mL, more preferably 0.1 to 500 mg/mL. There are no particular limitations on the concentration of the coating layer component (s) (e.g. a lipid) in the liquid C as long as the core fine particles can be coated therewith as disclosed in claim 1, and the coating layer component(s) concentration is preferably 1 μg/mL to 1 g/mL, more preferably 0.1 to 400 mg/mL. In the coated fine particles obtained by the method of the present invention, the weight ratio of the coating layer component (s) to the core fine particles is preferably 1:0.1 to 1:1000, more preferably 1:1 to 1:10.

The average particle diameter of the coated fine particles obtained by the method of the invention is preferably 300 nm or less, more preferably 200 nm or less, further preferably 130 nm or less, most preferably 115 nm or less, and for example, the size of the coated fine particles is preferably such that the coated fine particles can be administered by injection. The size of the obtained coated fine particles can be increased by increasing the size of the core fine particles to be coated, while the size of the coated fine particles can be reduced by reducing the size of the core fine particles.

The above coated fine particles may be modified with a protein such as an antibody, a saccharide, a glycolipid, an amino acid, a nucleic acid, a various low molecular compound, a polymer compound, or the like, and the coated fine particles of the present invention include thus-modified particles. The lipid membrane of the coated fine particles may be surface-modified with a protein such as an antibody, a peptide, a fatty acid compound, or the like, for example in order to apply the coated fine particles for targeting [edited by D. D. Lasic and F. Martin, "Stealth Liposome", United States, CRC Press Inc., 1995, p. 93-102]. Further, the coated fine particles may be surface-modified with a nonionic surfactant (having the same definition as above), a cationic surfactant (having the same definition as above), an anionic surfactant (having the same definition as above), a polymer (having the same definition as above), a polyoxyethylene derivative (having the same definition as above), or the like, as with the liposome used as a component of the fine particles. The coated fine particles of the invention include thus-surface-modified particles. Similarly, the coated fine particles may be surface-modified with a lipid derivative, a fatty acid derivative, or an aliphatic hydrocarbon derivative of a water-soluble substance; a lipid derivative or a fatty acid derivative of one or more substance(s) selected from a saccharide, a peptide, a nucleic acid, or a water-soluble polymer; a surfactant; or the like. The lipid derivative, fatty acid derivative, or aliphatic hydrocarbon derivative of a water-soluble substance, the lipid derivative or fatty acid derivative of a saccharide, a peptide, a nucleic acid, or a water-soluble polymer, and the surfactant, used for the surface modification, have the same definitions as the above-mentioned lipid derivative, fatty acid derivative, or aliphatic hydrocarbon derivative of a water-soluble substance, used as a component for the lipid membrane, and the above-described lipid derivative or fatty acid derivative of a saccharide, a peptide, a nucleic acid, or a water-soluble polymer, and the surfactant, used for preparing the core fine particles having the hydrophilic moiety on part of or on all the surfaces of the core fine particles, and the components of the lipid membrane include them.

The coated fine particles produced by the method of the present invention can be used, for example, as a pharmaceutical preparation intended for stabilizing a drug in a living body component such as blood component (e.g., blood or digestive fluid), reducing a side effect, increasing accumulation of a drug in a target organ of a tumor, etc., improving absorption of a drug in oral or transmucosal administration, or the like.

In a case where at least one constituent component of the core fine particles is a drug such as an antitumor drug or an anti-inflammatory drug, the coated fine particles produced by the method of the present invention can be used as a drug-comprising carrier for carrying the drug to a tumor site or an inflammation site, and thus the drug can be delivered to the tumor or inflammation site by administering the coated fine particles. When the drug is the antitumor drug, the coated fine particles can be administered as a tumor therapeutic agent for treating tumor. When the drug is the anti-inflammatory drug, the coated fine particles can be administered as a therapeutic agent for treating inflammation. A tissue or organ in which neovascularization is enhanced is one of the sites in which the coated fine particles produced by the method of the present invention can be delivered. When at least one constituent component of the coated fine particles is a therapeutic drug for a disease in the tissue or organ, the fine particles can be administered as a therapeutic agent for treating the disease.

In the case of using the coated fine particles produced by the method according to the present invention as a pharmaceutical preparation, the dispersion liquid of the coated fine particles prepared in the above manner may be used as it is, for example, in the form of an injection preparation, etc. The ratio of polar organic solvent in the dispersion liquid may be reduced by adding a solvent comprising a solvent other than the polar organic solvent contained in the dispersion liquid, mixable with the polar organic solvent, and/or by selectively removing the polar organic solvent by evaporation, semipermeable membrane separation, fractional distillation, or the like. Further, the dispersion liquid or the modified dispersion liquid having the ratio of the polar organic solvent reduced may be subjected to filtration, centrifugation, or the like to remove the solvents. The dispersion liquid or the dispersion liquid in which an excipient such as mannitol, lactose, trehalose, maltose, or glycine is added may be subjected to lyophilization when used as a pharmaceutical preparation.

In the case of an injection preparation, it is preferred that an injection preparation is prepared by mixing, for example, water, an acid, an alkali, any of various buffers, a physiological saline solution, an amino acid infusion or the like with the dispersion liquid of or the above-mentioned coated fine particles, dispersion liquid in which the ratio of the polar organic solvent is reduced or the above-mentioned coated fine particles obtained by removing the solvent or by lyophilization. Further, it is possible to prepare an injection preparation by adding an antioxidant such as citric acid, ascorbic acid, cysteine or EDTA, an isotonic agent such as glycerol, glucose, sodium chloride or the like. Further, it can also be cryopreserved by adding a cryopreservation agent such as glycerol.

Further, the coated fine particles produced by the method according to the present invention may be formulated into an oral preparation such as a capsule, a tablet or a granule by granulation along with an appropriate excipient or the like, drying or the like.

A kit may be used in the producing method of the invention. The kit may comprise at least the core fine particles, the coating layer component, and a liquid comprising the polar organic solvent, for preparing the coated fine particles by the method of the present invention. For example, the components for preparing the coated fine particles by the method of the present invention, such as the core fine particles, the coating layer component, and the liquid comprising the polar organic solvent, are filled in small vessels respectively, and the coated fine particles can be prepared by mixing the components according to the method of the invention. There are no limitations on the mixing, such as a limitation that the components must be gradually mixed, and thus the kit is advantageous in that the coated fine particles can be easily prepared.

The invention will be specifically described below with reference to Examples without intention of restricting the scope of the invention.

Example 1

Coating layer component(s) solution (liquid A); 60 mg of EPC (available from NOF Corporation, the same applies in the following examples) and 12.5 mg of PEG-DSPE (available from NOF Corporation, the same applies in the following examples) were dissolved in 2 mL of a mixed solvent of ethanol/water (8/5) to prepare a liquid A1.

Dispersion liquid of core fine particles (liquid B) ; 10 mg of dextran fluorescein anionic (FD, available from Molecular Probes, Inc., the same applies in the following examples), 60 mg of DOTAP (available from Avanti Polar Lipids, Inc. , the same applies in the following examples), and PEG-DSPE (24 mg) were dispersed in water (2 mL). The resulting dispersion liquid was passed through a polycarbonate membrane having a pore diameter of 0.1 $\mu$m (available from Whatman, Inc., the same applies in the following examples) 31 times to prepare a liquid B1.

The liquid B1 (0.5 mL) was added to the liquid A1 dropwise such that the ethanol concentration was approximately 50 vol% and the coating layer components and the core fine particles were dispersed at the concentration, to obtain coated fine particles dispersion liquid.

The obtained dispersion liquid was mixed with water (22.5 mL) and subjected to ultracentrifugation (1 hour, 110,000 $\times$ g, 25°C), and the supernatant was removed to obtain a preparation.

Example 2 (not according to the invention)

Dispersion liquid of coating layer component (s) (liquid D); EPC (60 mg) and PEG-DSPE (12.5 mg) were dispersed in 1.6 mL of a mixed solvent of ethanol/water (1/1) to prepare a liquid D2.

Dispersion liquid of core fine particles (liquid E) ; FD (10 mg), DOTAP (60 mg), and PEG-DSPE (24 mg) were dispersed in water (1 mL). The resulting dispersion liquid was passed through a polycarbonate membrane having a pore diameter of 0. 1 $\mu$m 31 times to prepare a core dispersion liquid. Ethanol (0.25 mL) was added to the obtained liquid (0.25 mL) such that the ethanol concentration was approximately 50 vol%, to prepare a liquid E2.

The liquid D2 was mixed with the liquid E2 such that the ethanol concentration was approximately 50 vol% and the coating layer components and the core fine particles were dispersed at the concentration, to obtain dispersion liquid of coated fine particles.

The obtained dispersion liquid of coated fine particles was mixed with water (23 mL) and subjected to ultracentrifugation in the same manner as Example 1, to obtain a preparation.

Example 3 (not according to the invention)

Dispersion liquid of coating layer components and core fine particles (liquid H); FD (10 mg), DOTAP (60 mg), and PEG-DSPE (24 mg) were dispersed in water (1mL). The resulting dispersion liquid was passed through a polycarbonate membrane having a pore diameter of 0.1 μm 31 times. Separately EPC (120 mg) and PEG-DSPE (25 mg) were dispersed in water (2 mL). The resulting dispersion liquid was passed through a polycarbonate membrane having a pore diameter of 0.4 μm (available from Whatman, Inc., the same applies in the following examples) 11 times, and passed through a polycarbonate membrane having a pore diameter of 0.1 μm 21 times. Thus-obtained dispersion liquid of FD, DOTAP, and PEG-DSPE was mixed with the dispersion liquid of EPC and PEG-DSPE to prepare a liquid H3.

Ethanol (1.25 mL) was added to the liquid H3 such that the ethanol concentration was approximately 50 vol% and the coating layer components and the core fine particles were dispersed at the concentration, to obtain a dispersion liquid of coated fine particles.

The obtained dispersion liquid of coated fine particles was mixed with water (22.5 mL) and subjected to ultracentrifugation in the same manner as Example 1, to obtain a preparation.

Comparative Example 1

Coating layer component (s) solution (liquid A); EPC (60 mg) and PEG-DSPE (12.5 mg) were dissolved in ethanol (1 mL) to prepare a liquid a1.

Dispersion liquid of core fine particles d (liquid B); FD (30 mg), DOTAP (180 mg), and PEG-DSPE (72 mg) were dispersed in water (9 mL). The resulting dispersion liquid was passed through a polycarbonate membrane having a pore diameter of 0. 1 μm 31 times to prepare a liquid b1.

The liquid a1 (0.25 mL) was mixed with the liquid b1 (0.75 mL) such that the ethanol concentration was approximately 62.5 vol%, and the coating layer components were dissolved and the core fine particles were dispersed at the concentration. Distillated water (23 mL) was added to the mixture at a rate of 1 mL/minute while stirring, to obtain dispersion liquid of coated fine particles.

The obtained dispersion liquid was subjected to ultracentrifugation in the same manner as Example 1, to obtain a preparation.

Test Example 1

The average particle diameter, recovery rates of FD and EPC, and stability in fetal bovine serum (FBS) of each of the preparations obtained in Examples 1 to 3 and Comparative Example 1 were examined by the following methods. The results are shown in Table 1.

Average particle diameter

The average particle diameter of the coated fine particles in a phosphate buffered saline (PBS) was measured by a dynamic light scattering method (DLS) using an apparatus A MODEL ELS-800 (manufactured by Otsuka Electronics, Co., Ltd., the same applies in the following examples).

Recovery rates of FD and EPC

Each preparation was diluted with PBS such that the total lipid concentration was 30 mg/mL, and was further diluted 1,000-fold with PBS. 50 μL of 10-w/v% TRITON X-100 (available from Wako Pure Chemical Industries, Ltd., the same applies in the following examples) and PBS (400 μL) were added to each of the diluted liquid (50 μL), and stirred by a vortex mixer. The supernatant obtained by the ultracentrifugation in each of above Examples and Comparative Examples was diluted 10-fold with PBS, and 10-w/v% TRITON X-100 (50 μL) and PBS (400 μL) were added to each of the diluted liquid (50 μL), and stirred by a vortex mixer. 100 μL of thus-obtained liquids were placed on a 96-well microplate respectively, and the fluorescence intensities thereof were measured at an excitation wavelength of 485 nm and a fluorescence wavelength of 530 nm using a fluorescence plate reader (ARVOsx-4 manufactured by Wallac, the same applies in the following examples). Meanwhile, the fluorescence intensities of FD-PBS liquids having concentrations of 1, 0.5, and 0.25 μg/mL were measured respectively, to obtain a calibration curve. The FD concentration of each preparation was obtained using the calibration curve. Further, the EPC concentration of each preparation was obtained by using Phospholipid C-TEST WAKO (available from Wako Pure Chemical Industries, Ltd., the same applies in the following examples).

The recovery rate of FD and recovery rate of EPC of each preparation were calculated using the following equations (1) and (2).

[Equation 1]

$$\text{Recovery rate of FD (\%)} = A_1 / (A_1 + B_1) \times 100 \qquad (1)$$

$A_1$: FD content of coated fine particles ($\mu$g) = Concentration of FD in coated fine particles fraction ($\mu$g/mL) $\times$ Volume of coated fine particles (mL)

$B_1$: FD content of supernatant ($\mu$g) = concentration of FD in supernatant ($\mu$g/mL) $\times$ Volume of supernatant (mL)

[Equation 2]

$$\text{Recovery rate of EPC (\%)} = A_2 / (A_2 + B_2) \times 100 \qquad (2)$$

$A_2$: EPC content of coated fine particles ($\mu$g) = Concentration of EPC in coated fine particles fraction ($\mu$g/mL) $\times$ Volume of coated fine particles (mL)

$B_2$: EPC content of supernatant ($\mu$g) = Concentration of EPC in supernatant ($\mu$g/mL) $\times$ Volume of supernatant (mL)

Stability in FBS

Each preparation was diluted with PBS such that the total lipid concentration was 30 mg/mL, and FBS (2970 $\mu$L) was added to and mixed with 30 $\mu$L of the diluted liquid.

500-$\mu$L portions were isolated from the obtained mixture immediately after the mixing and after the mixture was allowed to stand at 37°C for 3 hours, and subjected to a gel permeation chromatography (GPC), respectively. 10 fractions (each comprising 100 droplets) were recovered from each portion, and the fractions were stirred by shaking with a vortex mixer to prepare samples. To determine the FD content of each sample, 10 w/v% TRITON X-100 (50 $\mu$L) and PBS (400 $\mu$L) were added to 50 $\mu$L of the sample and stirred by a vortex mixer. 100 $\mu$L of thus-obtained liquids were placed on a 96-well microplate respectively, and the fluorescence intensities thereof were measured at an excitation wavelength of 485 nm and a fluorescence wavelength of 530 nm using ARVOsx-4. The drug retentions of the fine particles after 0 hour and after 3 hours were calculated using the following equation (3).

[Equation 3]

$$\text{FD retention after 0 hour (\%)} = A_3 / B_3 \times 100$$

$$\text{FD retention after 3 hours (\%)} = C_3 / D_3 \times 100 \qquad (3)$$

$A_3 = \Sigma\{$(Fluorescence intensity of fraction)Coated fine particles fractions (after 0 hour) $\times$ (Volume of fraction) Coated fine particles fractions (after 0 hour)$\}$

$B_3 = \Sigma\{$ (Fluorescence intensity of fraction)All fractions (after 0 hour) $\times$ (Volume of fraction) All fractions (after 0 hour)$\}$

$C_3 = \Sigma\{$(Fluorescence intensity of fraction)Coated fine particles fractions (after 3 hours) $\times$ (Volume of fraction) Coated fine particles fractions (after 3 hours)$\}$

$D_3 = \Sigma\{$ (Fluorescence intensity of fraction) All fractions (after 3 hours) $\times$ (Volume of fraction) All fractions (after 3 hours) $\}$

[Table 1]

| | Average particle diameter (nm) | Recovery rate (%) | | Retention in FBS (%) | |
|---|---|---|---|---|---|
| | | FD | EPC | 0 hour | 3 hours |
| Ex. 1 | 128 | 92 | 93 | Not measured | Not measured |
| Ex. 2 | 150 | 85 | 84 | 61 | 58 |
| Ex. 3 | 114 | 86 | 82 | 63 | 59 |
| Comp. Ex. 1 | 145 | 96 | 88 | 68 | 67 |

As shown in Table 1, the preparations obtained in Examples 1 to 3 had small particle diameters as with the preparation obtained in Comparative Example 1, and the preparations of Examples 1 and 3 had smaller particle diameters of 130 nm or less and 115 nm or less, respectively. Further, in the preparations of Examples 1 to 3, the drug was efficiently and firmly enclosed in the coated fine particles as the preparation of Comparative Example 1. The method of the present invention for producing the coated fine particles is simpler than conventional methods, and without a limitation that the components have to be gradually mixed, etc. Despite this, excellent coated fine particles similar to the ones obtained in the conventional methods can be obtained.

Example 4

PBS was added to the preparation of Example 1 to adjust the EPC concentration at 24 mg/mL, and 0.5 mL of the obtained liquid was mixed with a solution prepared by dissolving PEG-DSPE (2.5 mg) in ethanol (0.02mL). The mixture was heated at 70°C for 2 minutes to obtain a preparation surface-modified with PEG.

Comparative Example 2

PBS was added to the preparation of Comparative Example 1 to adjust the EPC concentration at 24 mg/mL, and 0.5 mL of the obtained liquid was mixed with a solution prepared by dissolving PEG-DSPE (2. 5 mg) in ethanol (0.02mL). The mixture was heated at 70°C for 2 minutes to obtain a preparation surface-modified with PEG.

Test Example 2

The blood kinetics of the preparations obtained in Example 4 and Comparative Example 2 were examined by the following methods. The results are shown in Table 2 and Fig. 1.

Blood kinetics

Each preparation was diluted with PBS such that the total lipid concentration was 5 mg/mL, and administered to a rat at a lipid/rat weight ratio of 10 mg/kg. The blood samples were collected from the rat 1 at minute, 10 minutes, 30 minutes, 1 hour, 3 hours, 6 hours, and 24 hours after the administration, and were subjected to centrifugation to obtain plasmas, respectively. To determine the FD content of each plasma, 10 w/v% TRITON X-100 (50 $\mu$L) and PBS (400 $\mu$L) were added to 50 $\mu$L of the plasma and stirred by a vortex mixer. 100 $\mu$L of thus-obtained liquids were placed on a 96-well microplate respectively, and the fluorescence intensities thereof were measured at an excitation wavelength of 485 nm and a fluorescence wavelength of 530 nm using ARVOsx-4. Meanwhile, the fluorescence intensities of FD-PBS liquids having concentrations of 1, 0.5, and 0.25 $\mu$g/mL were measured respectively, to obtain a calibration curve. The FD concentration of plasma was obtained using the calibration curve. The plasma amount per 100-g rat weight was considered as 7.8 mL, and thus the drug retention in blood (%) to the administration dose was calculated, and $AUC_{0.24h}$ ($\mu$g·min/dose) was calculated by trapezoidal method.

[Table 2]

|  | Average particle diameter (nm) | AUC ($\mu$g·min/dose) |
| --- | --- | --- |
| Ex. 4 | 128 | 423 |
| Comp. Ex. 2 | 145 | 342 |

As shown in Table 2, the preparation obtained in Example 4 exhibited an excellent retention in blood of a living body as the preparation obtained in Comparative Example 2. The method of the present invention for producing the coated fine particles is a simple method, and despite this, excellent coated fine particles similar to the ones obtained in the conventional methods.

Example 5

Coating layer component (s) solution (liquid A); EPC (15 mg) and PEG-DSPE (3.125 mg) were dissolved in 1 mL of a mixed solvent of ethanol/water (8/5) to prepare a liquid A5.

Dispersion liquid of core fine particles (liquid B); DOTAP (90 mg) and PEG-DSPE (36 mg) were dispersed in water (2 mL). The resulting dispersion liquid was passed through a polycarbonate membrane having a pore diameter of 0.4 $\mu$m 11 times, passed through a polycarbonate membrane having a pore diameter of 0. 1 $\mu$m 11 times, and passed through a polycarbonate membrane having a pore diameter of 0. 05 μm (available from Whatman, Inc. , the same applies in the following examples) 25 times. To 0.167 mL of the dispersion liquid was added a solution comprising 0.083 mL of water and 1.87 mg of ODN (fluorescein isothiocyanate-labeled, phosphorothioate oligodeoxyribonucleotide, having a sequence of (5') CCT CTT ACC TCA G (3'), a base number of 13, and a molecular weight of 4573.57, available from SciMedia Ltd. , the same applies in the following examples), to prepare a liquid B5.

The liquid A5 was mixed with the liquid B5 such that the ethanol concentration was approximately 50 vol% and the coating layer components and the core fine particles were dispersed at the concentration, to obtain a dispersion liquid of coated fine particles.

The obtained dispersion liquid of coated fine particles was mixed with water (11.25 mL) and subjected to ultracentrifugation (1 hour, 110,000 × g, 25°C), and the supernatant was removed to obtain a preparation.

Example 6

Coating layer component (s) solution (liquid A) ; EPC and PEG-DSPE were dissolved in 0.9 mL of a mixed solvent of ethanol/water (8/5), the amounts of EPC and PEG-DSPE being the same as those of the liquid A1 in Example 5, to prepare a liquid A6.

Dispersion liquid of core fine particles (liquid B); Distillated water (0.1 mL) was added to the liquid B5 of Example 5 to prepare a liquid B6.

The liquid A6 was mixed with the liquid B6 such that the ethanol concentration was approximately 45 vol% and the coating layer components and the core fine particles were dispersed at the concentration, to obtain a dispersion liquid of coated fine particles.

The obtained dispersion liquid of coated fine particles was mixed with water (10 mL) and subjected to ultracentrifugation in the same manner as Example 5, to obtain a preparation.

Example 7

Coating layer component (s) solution (liquid A); EPC and PEG-DSPE were dissolved in 0.8 mL of a mixed solvent of ethanol/water (8/5), the amounts of EPC and PEG-DSPE being the same as those of the liquid A5 in Example 5, to prepare a liquid A7.

Dispersion liquid of core fine particles (liquid B); Water (0.2 mL) was added to the liquid B5 of Example 5 to prepare a liquid B7.

The liquid A7 was mixed with the liquid B7 such that the ethanol concentration was approximately 40 vol% and the coating layer components and the core fine particles were dispersed at the concentration, to obtain a dispersion liquid of coated fine particles.

The obtained dispersion liquid of coated fine particles was mixed with water (8.75 mL) and subjected to ultracentrifugation in the same manner as Example 5, to obtain a preparation.

Example 8

Coating layer component (s) solution (liquid A); EPC and PEG-DSPE were dissolved in 0.7 mL of a mixed solvent of ethanol/water (8/5), the amounts of EPC and PEG-DSPE being the same as those of the liquid A5 in Example 5, to prepare a liquid A8.

Dispersion liquid of core fine particles (liquid B); Distillated water (0.3 mL) was added to the liquid B5 of Example 5 to prepare a liquid B8.

The liquid A8 was mixed with the liquid B8 such that the ethanol concentration was approximately 35 vol% and the coating layer components and the core fine particles were dispersed at the concentration, to obtain a dispersion liquid of coated fine particles.

The obtained dispersion liquid of coated fine particles was mixed with water (7.5 mL) and subjected to ultracentrifugation in the same manner as Example 5, to obtain a preparation.

Example 9 (not according to the invention)

Coating layer component(s) solution (liquid A) ; EPC and PEG-DSPE were dissolved in 0.5 mL of a mixed solvent of ethanol/water (8/5), the amounts of EPC and PEG-DSPE being the same as those of the liquid A5 in Example 5, to prepare a liquid A9.

Dispersion liquid of core fine particles (liquid B); Distillated water (0.5 mL) was added to the liquid B5 of Example 5 to prepare a liquid B9.

The liquid A9 was mixed with the liquid B9 such that the ethanol concentration was approximately 25 vol% and the coating layer components and the core fine particles were dispersed at the concentration, to obtain a dispersion liquid of coated fine particles.

The obtained dispersion liquid of coated fine particles was mixed with water (5 mL) and subjected to ultracentrifugation in the same manner as Example 5, to obtain a preparation.

Comparative Example 3

Coating layer component (s) solution (liquid A) ; EPC (30 mg) and PEG-DSPE (6.25 mg) were dissolved in ethanol (1 mL) to prepare a liquid a3.

Dispersion liquid of core fine particles (liquid B); DOTAP (60 mg) and PEG-DSPE (24 mg) were dispersed in water (2 mL). The resulting dispersion liquid was passed through a polycarbonate membrane having a pore diameter of 0.4 $\mu$m 11 times, passed through a polycarbonate membrane having a pore diameter of 0.1 $\mu$m 11 times, and passed through a polycarbonate membrane having a pore diameter of 0.05 $\mu$m 21 times. To 0.5 mL of the dispersion liquid was added a solution comprising 0.25 mL of water and 3.75 mg of ODN to prepare a liquid b3.

0.25 mL of the liquid a3 was mixed with the liquid b3 such that the ethanol concentration was approximately 62.5 vol%, and the coating layer components were dissolved and the core fine particles were dispersed at the concentration. Water (23 mL) was added to the mixture at a rate of 1 mL/minute while stirring, to obtain a dispersion liquid of coated fine particles.

The obtained dispersion liquid of coated fine particles was subjected to ultracentrifugation in the same manner as Example 5, to obtain a preparation.

Test Example 3

The average particle diameter, recovery rates of ODN and EPC, and stability in FBS of each of the preparations obtained in Examples 5 to 9 and Comparative Example 3 were examined by the following methods. The results are shown in Table 3.

Average particle diameter

The average particle diameter of the coated fine particles in PBS was measured by DLS.

Recovery rates of ODN and EPC

Each preparation was diluted with PBS such that the total lipid concentration was 30 mg/mL, and was further diluted 1, 000-fold with PBS. 10-w/v% TRITON X-100 (50 $\mu$L) and PBS (400 $\mu$L) were added to 50 $\mu$L of the diluted liquid, and stirred by a vortex mixer. The supernatant obtained by the ultracentrifugation in each of above Examples and Comparative Example was diluted 10-fold with PBS, and 10-w/v% TRITON X-100 (50 $\mu$L) and PBS (400 $\mu$L) were added to 50 $\mu$L of the diluted liquid, and stirred by a vortex mixer. 100 $\mu$L of thus-obtained liquids were placed on a 96-well microplate respectively, and the fluorescence intensities thereof were measured at an excitation wavelength of 485 nm and a fluorescence wavelength of 530 nm using ARVOsx-4. Meanwhile, the fluorescence intensities of ODN-PBS liquids having concentrations of 1.5, 1.5/4, $1.5/4^2$, $1.5/4^3$, $1.5/4^4$ and $1.5/4^5$ $\mu$g/mL were measured respectively, to obtain a calibration curve. The ODN concentration of each preparation was obtained using the calibration curve. Further, the EPC concentration of each preparation was obtained by using Phospholipid C-TEST WAKO.

The recovery rate of ODN and recovery rate of EPC of each preparation were calculated using the following equations (4) and (5).

[Equation 4]

$$\text{Recovery rate of ODN (\%)} = A_4 \, / \, (A_4 + B_4) \times 100 \quad (4)$$

$A_4$: ODN content of coated fine particles ($\mu$g) = Concentration of ODN in coated fine particles ($\mu$g/mL) $\times$ Volume of coated fine particles (mL)

$B_4$: ODN content of supernatant ($\mu$g) = Concentration of ODN in supernatant ($\mu$g/mL) $\times$ Volume of supernatant (mL)

[Equation 5]

$$\text{Recovery rate of EPC (\%)} = A_2 / (A_5 + B_5) \times 100 \quad (5)$$

$A_5$: EPC content of coated fine particles ($\mu$g) = Concentration of EPC in coated fine particles ($\mu$g/mL) $\times$ Volume of coated fine particles (mL)

$B_5$: EPC content of supernatant ($\mu$g) = Concentration of EPC in supernatant ($\mu$g/mL) $\times$ Volume of supernatant (mL)

Stability in FBS

Each preparation was diluted with PBS such that the total lipid concentration was 30 mg/mL, and 50-mg/mL dextran sodium sulfate (60 $\mu$L) and FBS (2910 $\mu$L) were added to and mixed with 30 $\mu$L of the diluted liquid.

500-$\mu$L portions were isolated from the obtained mixture immediately after the mixing and after the mixture was allowed to stand at 37°C for 3 hours, and subjected to GPC, respectively. 10 fractions (each comprising 100 droplets) were recovered from each portion, and the fractions were stirred by shaking with a vortex mixer to prepare samples. To determine the ODN content of each sample, 10 w/v% TRITON X-100 (50 $\mu$L) and PBS (400 $\mu$L) were added to 50 $\mu$L of the sample and stirred by a vortex mixer. 100 $\mu$L of thus-obtained liquids were placed on a 96-well microplate respectively, and the fluorescence intensities thereof were measured at an excitation wavelength of 485 nm and a fluorescence wavelength of 530 nm using ARVOsx-4. The recovery rates of the drug after 0 hour and after 3 hours were calculated using the following equation (6).

[Equation 6]

$$\text{Recovery rate of ODN after 0 hour (\%)} = A_6 / B_6 \times 100$$

$$(6)$$

$$\text{Recovery rate of ODN after 3 hours (\%)} = C_6 / D_6 \times 100$$

$A_6 = \Sigma\{(\text{Fluorescence intensity of fraction})_{\text{Coated fine particles fractions (after 0 hour)}} \times (\text{Volume of fraction})_{\text{Coated fine particles fractions (after 0 hour)}}\}$

$B_6 = \Sigma\{(\text{Fluorescence intensity of fraction})_{\text{All fractions (after 0 hour)}} \times (\text{Volume of fraction})_{\text{All fractions (after 0 hour)}}\}$

$C_6 = \Sigma\{(\text{Fluorescence intensity of fraction})_{\text{Coated fine particles fractions (after 3 hours)}} \times (\text{Volume of fraction})_{\text{Coated fine particles fractions (after 3 hours)}}\}$

$D_6 = \Sigma\{(\text{Fluorescence intensity of fraction})_{\text{All fractions (after 3 hours)}} \times (\text{Volume of fraction})_{\text{All fractions (after 3 hours)}}\}$

[Table 3]

| | Average particle diameter (nm) | Recovery rate (%) | | Retention in FBS (%) | |
|---|---|---|---|---|---|
| | | ODN | Lipid | 0 hour | 3 hours |
| Ex. 5 | 124 | 84 | 45 | 68 | 43 |
| Ex. 6 | 125 | 85 | 57 | 84 | 62 |
| Ex. 7 | 119 | 88 | 70 | 87 | 73 |
| Ex. 8 | 129 | 88 | 70 | 79 | 61 |
| Ex. 9 | 110 | 85 | 29 | 55 | 32 |
| Comp. Ex. 3 | 145 | 97 | 59 | Not measured | Not measured |

As shown in Table 3, the preparations obtained in Examples 5 to 9 had small particle diameters as the preparation obtained in Comparative Example 3, and the preparations of Examples 5 to 8 had smaller particle diameters of 130 nm or less, respectively and the preparation of Example 9 had a smaller particle diameter of 115 nm or less. Further, in the preparations of Examples 5 to 9, the drug was efficiently and firmly enclosed in the coated fine particles as the preparation of Comparative Example 3. The method of the invention carried out in Examples 5 to 9 comprised the steps of: preparing the liquid A comprising the polar organic solvent, in which the coating layer components were dissolved; preparing the liquid B mixable with the liquid A, in which the core fine particles were dispersed and the polar organic solvent was not contained or the polar organic solvent was contained in a ratio lower than that in the liquid A; and mixing the liquid A with the liquid B to prepare the liquid C, thereby producing the coated fine particles comprising the core fine particles coated with the coating layer components. The method is simpler than conventional methods, and without a limitation that the components have to be gradually mixed, etc. Despite this, excellent coated fine particles could be obtained by the method of the present invention similar to the ones obtained in the conventional methods, even in the case of using the relatively large drug of ODN as the core fine particles.

Example 10 (not according to the invention)

Dispersion liquid of coating layer components and core fine particles (liquid H); EPC (30 mg) and PEG-DSPE (6.25 mg) were dispersed in water (0.75 mL). The resulting dispersion liquid was passed through a polycarbonate membrane having a pore diameter of 0.4 $\mu$m 11 times, and passed through a polycarbonate membrane having a pore diameter of 0.1 $\mu$m 25 times, to obtain a lipid dispersion liquid. 0.375 mL of the lipid dispersion liquid was mixed with the liquid B5 of Example 5 to prepare a liquid H10.

Ethanol (0.625 mL) was added to the liquid H10 such that the ethanol concentration was approximately 50 vol% and the coating layer components and the core fine particles were dispersed at the concentration. The obtained liquid was stirred for 30 minutes to obtain a dispersion liquid of coated fine particles.

The obtained dispersion liquid of coated fine particles was mixed with water (11.25 mL) and subjected to ultracentrifugation in the same manner as Example 5, to obtain a preparation.

Example 11 (not according to the invention)

Dispersion liquid of coating layer components and core fine particles (liquid H); A liquid H11 was prepared in the same manner as the liquid H10 of Example 10 except that the amount of water was 1 mL and 0.5 mL of the lipid dispersion liquid was mixed with the liquid B5 of Example 5.

Ethanol (0.5 mL) was added to the liquid H11 such that the ethanol concentration was approximately 40 vol% and the coating layer components and the core fine particles were dispersed at the concentration. The obtained liquid was stirred for 30 minutes to obtain a dispersion liquid of coated fine particles.

The obtained dispersion liquid of coated fine particles was mixed with water (8.75 mL) and subjected to ultracentrifugation in the same manner as Example 5, to obtain a preparation.

Example 12 (not according to the invention)

Dispersion liquid of coating layer components and core fine particles (liquid H); A liquid H12 was prepared in the same manner as the liquid H10 of Example 10 except that the amount of water was 1. 125 mL and 0. 563 mL of the lipid dispersion liquid was mixed with the liquid B5 of Example 5.

Ethanol (0.438 mL) was added to the liquid H12 such that the ethanol concentration was approximately 35 vol% and the coating layer components and the core fine particles were dispersed at the concentration. The obtained liquid was stirred for 30 minutes to obtain a dispersion liquid of coated fine particles.

The obtained dispersion liquid of coated fine particles was mixed with water (7.5 mL) and subjected to ultracentrifugation in the same manner as Example 5, to obtain a preparation.

Example 13 (not according to the invention)

Dispersion liquid of coating layer components and core fine particles (liquid H); A liquid H13 was prepared in the same manner as the liquid H10 of Example 10 except that the amount of water was 1. 375 mL and 0. 688 mL of the lipid dispersion liquid was mixed with the liquid B5 of Example 5.

Ethanol (0.313 mL) was added to the liquid H13 such that the ethanol concentration was approximately 25 vol% and the coating layer components and the core fine particles were dispersed at the concentration. The obtained liquid was stirred for 30 minutes to obtain a dispersion liquid of coated fine particles.

The obtained dispersion liquid of coated fine particles was mixed with water (5 mL) and subjected to ultracentrifugation in the same manner as Example 5, to obtain a preparation.

Test Example 4

The average particle diameter, recovery rates of ODN and EPC, and stability in FBS of each of the preparations obtained in Examples 10 to 13 were examined in the same manner as Test Example 3. The results are shown in Table 4.

[Table 4]

| | Average particle diameter (nm) | Recovery rate (%) | | Retention in plasma (%) | |
|---|---|---|---|---|---|
| | | ODN | EPC | 0 hour | 3 hours |
| Ex. 10 | 108 | 79 | 43 | 56 | 29 |
| Ex. 11 | 98 | 90 | 76 | 82 | 61 |
| Ex. 12 | 99 | 93 | 74 | 88 | 70 |
| Ex. 13 | 95 | 90 | 79 | 73 | 60 |
| Comp. Ex. 3 | 145 | 97 | 59 | Not measured | Not measured |

As shown in Table 4, the preparations obtained in Examples 10 to 13 had small particle diameters as the preparation obtained in Comparative Example 3, and the preparation of Example 10 had a smaller particle diameter of 115 nm or less and the preparations of Examples 11 to 13 had smaller particle diameters of 100 nm or less, respectively. Further, in the preparations of Examples 10 to 13, the drug was efficiently and firmly enclosed in the coated fine particles as the preparation of Comparative Example 3. The method of the present invention carried out in example 13 comprised the steps of: preparing the liquid H, in which the coating layer components and the core fine particles were dispersed; and mixing the liquid H with the liquid mixable with the liquid H, in which the polar organic solvent was contained, to prepare the liquid I, thereby producing the coated fine particles comprising the core fine particles coated with the coating layer components. The method is simpler than conventional methods, and without a limitation that the components have to be gradually mixed, etc. Despite this, excellent coated fine particles could be obtained by the method of the present invention, similar to the ones obtained in conventional methods, even in the case of using the relatively large drug of ODN in the core fine particles.

As is clear from Tables 3 and 4, the method of the present invention of producing coated fine particles is advantageous also in that the amount of the polar organic solvent used can be smaller as compared with conventional methods.

Industrial Applicability

According to the present invention, there are provided a method for easily producing coated fine particles in which core fine particles are coated with a coating layer component, and the like. Further, there are provided a method of producing a coated fine particles in which a drug is efficiently and/or firmly enclosed.

**Claims**

1. A method of producing coated fine particles in which core fine particles are coated with a coating layer component, which comprises the steps of:

    preparing a liquid A comprising a coating layer component or coating layer components dissolved therein and a polar organic solvent;
    preparing a liquid B mixable with liquid A, in which the core fine particles are dispersed, and wherein a polar organic solvent is not contained, or said polar organic solvent is contained in a ratio lower than that in the liquid A; and
    mixing the liquid A with the liquid B to prepare a liquid C so that the concentration of the polar organic solvent in the liquid C is 30 to 60 vol%, the core fine particles are not dissolved in the liquid C and the coating layer component or components is/are in the dispersed state in the liquid C; and
    leaving or mixing the liquid C for 3 seconds to 30 minutes.

2. The method according to claim 1, wherein the coating layer component or components is/are one or more substance or substances selected from lipids and surfactants for forming a coating layer of a lipid membrane.

3. The method according to claim 1, wherein the coating layer component or components is/are one or more substance or substances selected from lipids and surfactants for forming a coating layer of a lipid membrane, and a lipid derivative or a fatty acid derivative of one or more substance or substances selected from saccharides, peptides, nucleic acids and water-soluble polymers, or a surfactant.

**4.** The method according to any one of claims 1 to 3, wherein the core fine particles comprise a lipid derivative or a fatty acid derivative of one or more substance or substances selected from saccharides, peptides, nucleic acids and water-soluble polymers, or a surfactant.

**5.** The method according to claim 3 or 4, wherein the lipid derivative or fatty acid derivative of one or more substance or substances selected from saccharides, peptides, nucleic acids and water-soluble polymers, or a surfactant is one or more substance or substances selected from polyethylene glycolated lipids, polyglycerin-modified lipids, polyethylene glycol alkyl ethers, polyethylene glycol sorbitan fatty acid esters, polyethylene glycol fatty acid esters, polyglycerin fatty acid esters, polyoxyethylene polyoxypropylene glycols and glycerin fatty acid esters.

**6.** The method according to any one of claims 1 to 5, wherein the core fine particles are fine particles comprising, as a constituent component, one or more component or components selected from a drug, a lipid assembly, liposome, emulsion particles, a polymer, a metal colloid and fine particles preparation.

**7.** The method according to any one of claims 1 to 5, wherein the core fine particles are fine particles comprising a drug as a constituent component.

**8.** The method according to any one of claims 1 to 5, wherein the core fine particles are fine particles comprising, as a constituent component, a complex of a drug and one or more component or components selected from a lipid assembly, liposome, emulsion particles, a polymer, a metal colloid and fine particles preparation.

**9.** The method according to claim 8, wherein the lipid assembly, the liposome, the emulsion particles, the polymer, the metal colloid or the fine particles preparation has an electrostatic charge opposite to that of the drug.

**10.** The method according to any one of claims 1 to 5, wherein the core fine particles are fine particles comprising, as a constituent component, a complex of an anionic drug and liposome comprising a cationic lipid.

**11.** The method according to any one of claims 1 to 5, wherein the core fine particles are fine particles comprising, as a constituent component, a complex of a cationic drug and liposome comprising an anionic lipid.

**12.** The method according to any one of claims 9 to 11, wherein the core fine particles are fine particles further comprising an adhesion-competitive agent.

**13.** The method according to any one of claims 6 to 12, wherein the drug is one or more component or components selected from peptides, proteins, nucleic acids, low-molecular compounds, saccharides and polymer compounds.

**14.** The method according to any one of claims 6 to 12, wherein the drug is one or more component or components selected from genes, DNAs, RNAs, oligonucleotides, plasmids and siRNAs.

**Patentansprüche**

**1.** Verfahren zur Herstellung beschichteter feiner Teilchen, in dem feine Kernteilchen mit einer Deckschichtkomponente beschichtet werden, umfassend die Schritte:

Herstellen einer Flüssigkeit A, umfassend eine darin gelöste Deckschichtkomponente oder Deckschichtkomponenten und ein polares organisches Lösungsmittel;
Herstellen einer Flüssigkeit B, die mit Flüssigkeit A mischbar ist, in der die feinen Kernteilchen dispergiert sind, und wobei kein polares organisches Lösungsmittel enthalten ist oder das polare organische Lösungsmittel in einem Verhältnis, das kleiner ist als das in der Flüssigkeit A, enthalten ist; und
Mischen der Flüssigkeit A mit der Flüssigkeit B zur Herstellung einer Flüssigkeit C, so dass die Konzentration des polaren organischen Lösungsmittels in der Flüssigkeit C 30 bis 60 Vol.-% beträgt, die feinen Kernteilchen nicht in der Flüssigkeit C gelöst werden und die Deckschichtkomponente oder -komponenten im dispergierten Zustand in der Flüssigkeit C vorliegt/vorliegen; und
Stehenlassen oder Mischen der Flüssigkeit C für 3 Sekunden bis 30 Minuten.

**2.** Verfahren nach Anspruch 1, wobei die Deckschichtkomponente oder -komponenten eine oder mehrere Substanz oder Substanzen, ausgewählt aus Lipiden und oberflächenaktiven Mitteln zum Bilden einer Deckschicht aus einer Lipidmembran, ist/sind.

3. Verfahren nach Anspruch 1, wobei die Deckschichtkomponente oder -komponenten eine oder mehrere Substanz oder Substanzen, ausgewählt aus Lipiden und oberflächenaktiven Mitteln zum Bilden einer Deckschicht aus einer Lipidmembran, und ein Lipidderivat oder ein Fettsäurederivat von einer oder mehreren Substanz oder Substanzen, ausgewählt aus Sacchariden, Peptiden, Nucleinsäuren und wasserlöslichen Polymeren, oder ein oberflächenaktives Mittel ist/sind.

4. Verfahren nach einem der Ansprüche 1 bis 3, wobei die feinen Kernteilchen ein Lipidderivat oder ein Fettsäurederivat von einer oder mehreren Substanz oder Substanzen, ausgewählt aus Sacchariden, Peptiden, Nucleinsäuren und wasserlöslichen Polymeren, oder ein oberflächenaktives Mittel umfassen.

5. Verfahren nach Anspruch 3 oder 4, wobei das Lipidderivat oder das Fettsäurederivat von einer oder mehreren Substanz oder Substanzen, ausgewählt aus Sacchariden, Peptiden, Nucleinsäuren und wasserlöslichen Polymeren, oder das oberflächenaktive Mittel eine oder mehrere Substanz oder Substanzen, ausgewählt aus Polyethylenglycolierten Lipiden, Polyglycerin-modifizierten Lipiden, Polyethylenglycolalkylethern, Polyethylenglycolsorbitan-Fettsäureestern, Polyethylenglycol-Fettsäureestern, Polyglycerin-Fettsäureestern, Polyoxyethylenpolyoxypropylenglycolen und Glycerin-Fettsäureestern, ist/sind.

6. Verfahren nach einem der Ansprüche 1 bis 5, wobei die feinen Kernteilchen feine Teilchen sind, die als Bestandteil eine oder mehrere Komponente oder Komponenten, ausgewählt aus einem Arzneimittel, einer Lipidanordnung, Liposom, Emulsionsteilchen, einem Polymer, einem Metallkolloid und Feinteilchenpräparat, umfassen.

7. Verfahren nach einem der Ansprüche 1 bis 5, wobei die feinen Kernteilchen feine Teilchen sind, die ein Arzneimittel als einen Bestandteil umfassen.

8. Verfahren nach einem der Ansprüche 1 bis 5, wobei die feinen Kernteilchen feine Teilchen sind, die als einen Bestandteil einen Komplex aus einem Arzneimittel und einer oder mehreren Komponente oder Komponenten, ausgewählt aus einer Lipidanordnung, Liposom, Emulsionsteilchen, einem Polymer, einem Metallkolloid und Feinteilchenpräparat, umfassen.

9. Verfahren nach Anspruch 8, wobei die Lipidanordnung, das Liposom, die Emulsionsteilchen, das Polymer, das Metallkolloid oder das Feinteilchenpräparat eine elektrostatische Ladung entgegengesetzt zu der des Arzneimittels aufweisen.

10. Verfahren nach einem der Ansprüche 1 bis 5, wobei die feinen Kernteilchen feine Teilchen sind, die als einen Bestandteil einen Komplex aus einem anionischen Arzneimittel und Liposom, umfassend ein kationisches Lipid, umfassen.

11. Verfahren nach einem der Ansprüche 1 bis 5, wobei die feinen Kernteilchen feine Teilchen sind, die als einen Bestandteil einen Komplex aus einem kationischen Arzneimittel und Liposom, umfassend ein anionisches Lipid, umfassen.

12. Verfahren nach einem der Ansprüche 9 bis 11, wobei die feinen Kernteilchen feine Teilchen sind, die ferner ein um die Adhäsion konkurrierendes Mittel umfassen.

13. Verfahren nach einem der Ansprüche 6 bis 12, wobei das Arzneimittel eine oder mehrere Komponente oder Komponenten, ausgewählt aus Peptiden, Proteinen, Nucleinsäuren, niedermolekularen Verbindungen, Sacchariden und Polymerverbindungen, ist.

14. Verfahren nach einem der Ansprüche 6 bis 12, wobei das Arzneimittel eine oder mehrere Komponente oder Komponenten, ausgewählt aus Genen, DNAs, RNAs, Oligonucleotiden, Plasmiden und siRNAs, ist.

**Revendications**

1. Procédé de production de particules fines revêtues dans lequel des particules fines de noyau sont revêtues d'un composant de couche de revêtement, qui comprend les étapes consistant à :

préparer un liquide A comprenant un composant de couche de revêtement ou des composants de couche de revêtement, dissous en son sein et un solvant organique polaire ;

préparer un liquide B pouvant être mélangé au liquide A, dans lequel les particules fines de noyau sont dispersées, et dans lequel un solvant organique polaire n'est pas contenu, ou ledit solvant organique polaire est contenu dans un rapport inférieur à celui qui est dans le liquide A ; et

mélanger le liquide A au liquide B pour préparer un liquide C de sorte que la concentration du solvant organique polaire dans le liquide C va de 30 à 60 % en volume, les particules fines de noyau ne sont pas dissoutes dans le liquide C et le composant ou les composants de couche de revêtement est/sont dans l'état dispersé dans le liquide C ; et

laisser ou mélanger le liquide C pendant 3 secondes à 30 minutes.

2. Procédé selon la revendication 1, dans lequel le composant ou les composants de couche de revêtement est/sont une substance ou plusieurs substances choisie(s) parmi des lipides et des agents tensioactifs pour former une couche de revêtement d'une membrane lipidique.

3. Procédé selon la revendication 1, dans lequel le composant ou les composants de couche de revêtement est/sont une substance ou plusieurs substances choisie(s) parmi des lipides et des agents tensioactifs pour former une couche de revêtement d'une membrane lipidique, et un dérivé de lipide ou un dérivé d'acide gras d'une substance ou de plusieurs substances choisie(s) parmi des saccharides, des peptides, des acides nucléiques et des polymères hydrosolubles, ou un agent tensioactif.

4. Procédé selon l'une quelconque des revendications 1 à 3, dans lequel les particules fines de noyau comprennent un dérivé de lipide ou un dérivé d'acide gras d'une substance ou de plusieurs substances choisie(s) parmi des saccharides, des peptides, des acides nucléiques et des polymères hydrosolubles, ou un agent tensioactif.

5. Procédé selon la revendication 3 ou 4, dans lequel le dérivé de lipide ou le dérivé d'acide gras d'une substance ou de plusieurs substances choisie(s) parmi des saccharides, des peptides, des acides nucléiques et des polymères hydrosolubles ou un agent tensioactif, est une substance ou plusieurs substances choisie(s) parmi des lipides polyéthylène glycolés, des lipides à modification polyglycérine, des éthers alkyliques de polyéthylène glycol, des esters d'acide gras de polyéthylène glycol sorbitan, des esters d'acide gras de polyéthylène glycol, des esters d'acide gras de polyglycérine, des esters d'acide gras de polyoxyéthylène polyoxypropylène glycols et de glycérine.

6. Procédé selon l'une quelconque des revendications 1 à 5, dans lequel les particules fines de noyau sont des particules fines comprenant, en tant que composant constitutif, un composant ou plusieurs composants choisi(s) parmi un médicament, un ensemble lipidique, un liposome, des particules d'émulsion, un polymère, un colloïde de métal et une préparation de particules fines.

7. Procédé selon l'une quelconque des revendications 1 à 5, dans lequel les particules fines de noyau sont des particules fines comprenant un médicament en tant que composant constitutif.

8. Procédé selon l'une quelconque des revendications 1 à 5, dans lequel les particules fines de noyau sont des particules fines comprenant, en tant que composant constitutif, un complexe d'un médicament et d'un composant ou plusieurs composants choisi(s) parmi un ensemble lipidique, un liposome, des particules d'émulsion, un polymère, un colloïde de métal et une préparation de particules fines.

9. Procédé selon la revendication 8, dans lequel l'ensemble lipidique, le liposome, les particules d'émulsion, le polymère, le colloïde de métal ou la préparation de particules fines possèdent une charge électrostatique opposée à celle du médicament.

10. Procédé selon l'une quelconque des revendications 1 à 5, dans lequel les particules fines de noyau sont des particules fines comprenant, en tant que composant constitutif, un complexe d'un médicament anionique et d'un liposome comprenant un lipide cationique.

11. Procédé selon l'une quelconque des revendications 1 à 5, dans lequel les particules fines de noyau sont des particules fines comprenant, en tant que composant constitutif, un complexe d'un médicament cationique et d'un liposome comprenant un lipide anionique.

12. Procédé selon l'une quelconque des revendications 9 à 11, dans lequel les particules fines de noyau sont des particules fines comprenant en outre un agent à adhésion concurrentielle.

13. Procédé selon l'une quelconque des revendications 6 à 12, dans lequel le médicament est un composant ou plusieurs composants choisi(s) parmi des peptides, des protéines, des acides nucléiques, des composés à faible masse moléculaire, des saccharides et des composés polymères.

14. Procédé selon l'une quelconque des revendications 6 à 12, dans lequel le médicament est un composant ou plusieurs composants choisi(s) parmi des gènes, des ADN, des ARN, des oligonucléotides, des plasmides et des ARNic.

Figure 1

EP 1 852 178 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0228367 A
- JP 2002508765 PCT
- JP 2003535832 PCT
- EP 1323415 A1
- JP 2572554 B
- JP 2659136 B

### Non-patent literature cited in the description

- **BANGHAM et al.** Journal of Molecular Biology. *J. Mol. Biol.,* 1965, vol. 13, 238-252
- Journal of Cell Biology. *J. Cell Biol.,* 1975, vol. 66, 621-634
- *FEBS Letters,* 1979, vol. 99, 210-214
- *Archives of Biochemistry and Biophysics,* 1981, vol. 212, 186-194
- *Proceedings of the National Academy of Science United States of America,* 1978, vol. 75, 4194-4198
- Stealth Liposome. CRC Press Inc, 1995, 93-102
- Emulsion and Nanosuspensions for the Formulation of Poorly Soluble Drugs. Scientific Publishers, 1998, 267-294